# EUROPEAN PATENT APPLICATION

(11) **EP 4 289 434 A1**
(43) Date of publication of application: **13.12.2023**
(21) Application number: 22749718.7
(22) Date of filing: 02.02.2022
(51) Int. Cl.: A61K 31/7034, A61K 8/60, A61K 31/7048, A61P 17/16, A61Q 19/00, C07H 15/203, C07H 17/07

(54) **BARRIER FUNCTION IMPROVER**

(30) Priority: 03.02.2021 JP 2021016111
(71) Applicant: Resonac Corporation, Tokyo 105-8518 (JP)
(72) Inventor: NAKAGAMI Yuko, Tokyo 105-8518 (JP)
(74) Representative: Strehl Schübel-Hopf & Partner
(86) International application number: PCT/JP2022/003962
(87) International publication number: WO 2022/168846

(57) **Abstract**

A barrier function improver containing methyl hesperidin as an active ingredient, and a barrier-function-improving composition containing the barrier function improver and a pharmaceutically acceptable carrier are provided.

## Description

### [Technical Field]

The present invention relates to a barrier function improver and a barrier-function-improving composition.

Priority is claimed on Japanese Patent Application No. 2021-016111, filed February 3, 2021, the content of which is incorporated herein by reference.

### [Background Art]

Examples of one of the most important physiological functions of the epidermis include a defensive function against dryness, ultraviolet rays, and other physical and chemical stimuli from the outside, and the stratum corneum plays a role of this defensive function. Corneocytes forming this stratum corneum are formed through a differentiation process called turnover of epidermal keratinocytes. When this differentiation of the skin is inhibited or abnormally accelerated for some reason, normal skin turnover is not performed, causing a deterioration of the barrier function (Non Patent Documents 1 and 2).

The deterioration of the barrier function of the skin increases transepidermal water loss, which causes rough skin, desquamation, itching, and the like, thereby causing so-called dry skin. A reduction or compositional change in intercellular lipids including stratum corneum ceramides has been reported in patients with aging skin, atopic dermatitis accompanying a barrier disorder, and psoriasis (Non Patent Documents 3 and 4).

In addition, amino acids associated with the water retention of the stratum corneum are decomposition products of a filaggrin protein of the cells of the stratum granulosum. In atopic dermatitis with strong dryness symptoms, it is known that profilaggrin, which is a filaggrin precursor, is reduced in the stratum corneum. A reduction in filaggrin causes a deterioration of the water retention ability of the stratum corneum and dry skin caused thereby. It is widely known that filaggrin is also important for maintaining and improving the skin barrier function (Non Patent Documents 5 and 6).

As a factor called a differentiation marker associated with the process of normal skin turnover and barrier formation, involucrin, loricrin, and transglutaminase 1 are known in addition to the above-mentioned filaggrin. In addition, aquaporin 3 (AQP3), which is abundant in skin keratinocytes, is important in improving the physiological barrier function of the skin by promoting water and glycerol transport (Non Patent Document 7). Furthermore, it has also been suggested that the expression of aquaporin 3 is significantly reduced at the site of skin inflammation, which is closely connected with the formation of dryness symptoms associated with various skin diseases (Patent Document 1).

In addition, because the skin barrier function is disrupted when tight junction constituent proteins present in the epidermal stratum granulosum are deleted at the genetic level, it is known that tight junctions also play an important role in the skin barrier function (Non Patent Document 8). When proteins, such as claudin 1 and occludin, which constitute tight junctions are reduced for some reason, a function as a permeation barrier for structural substances deteriorates, thereby causing skin symptoms such as dry skin, rough skin, atopic dermatitis, and various infectious diseases.

### [Citation List]

### [Patent Document]

[Patent Document 1]
Japanese Unexamined Patent Application, First Publication No. 2011-32191

### [Non Patent Documents]

[Non Patent Document 1]
   Mina Yaar et al., Retinoic Acid Delays the Terminal Differentiation of Keratinocytes in Suspension Culture. J Invest Dermatol 76:363-366, 1981.
[Non Patent Document 2]
   Akemi Ishida-Yamamoto et al., Structural organization of cornified cell envelopes and alterations in inherited skin disorders. Exp Dermatol 1998: 7: 1-10.
[Non Patent Document 3]
   Kayoko Akimoto et al., Quantitative Analysis of Stratum Corneum Lipids in Xerosis and Asteatotic Eczema. The Journal of Dermatology Vol.20: 1-6, 1993.
[Non Patent Document 4]
   Genji Imokawa et al., Decreased level of ceramides in stratum corneum of atopic dermatitis: an etiologic factor in atopic dry skin?. J Invest Dermatol 96:523-526, 1991.
[Non Patent Document 5]
   Tokuji Seguchi et al., Decreased expression of filaggrin in atopic skin. Arch Dermatol Res 288, 442-446, 1996.
[Non Patent Document 6]
   Hiroshi FUJITA et al., "Palo Azul Prevents Dryness-Induced Decrease in Filaggrin and Amino Acids", J. Soc. Cosmet. Chem. Jpn. 37(2), 84-91, 2003.
[Non Patent Document 7]
   Mariko Hara-Chikuma et al., Aquaporin-3 functions as a glycerol transporter in mammalian skin. Biol. Cell (2005) 97, 479-486.
[Non Patent Document 8]
   Takuo Yuki et al., Tight junction proteins in keratinocytes: localization and contribution to barrier function. Experimental Dermatology 16, 324-330, 2007.

### [Summary of Invention]

### [Technical Problem]

As described above, it is known that a skin barrier function deteriorates in rough skin, dry skin, psoriasis, atopy, and the like, and a drug capable of effectively improving the barrier function is demanded. However, it can be said that the effects of conventionally known drugs are still insufficient.

Therefore, an object of the present invention is to provide a barrier function improver capable of effectively improving a barrier function, and a barrier-function-improving composition containing the above-mentioned barrier function improver.

### [Solution to Problem]

The present invention includes the following aspects.
[1] A barrier function improver for skin containing: methyl hesperidin as an active ingredient.
[2] The barrier function improver according to [1], in which the methyl hesperidin is one or more selected from the group consisting of chalcone form methyl hesperidin represented by General Formula (1) and flavanone form methyl hesperidin represented by General Formula (2). 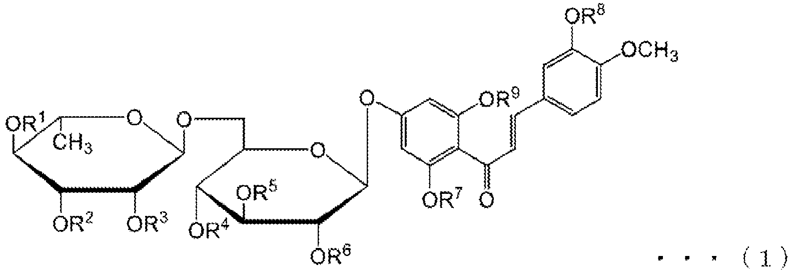 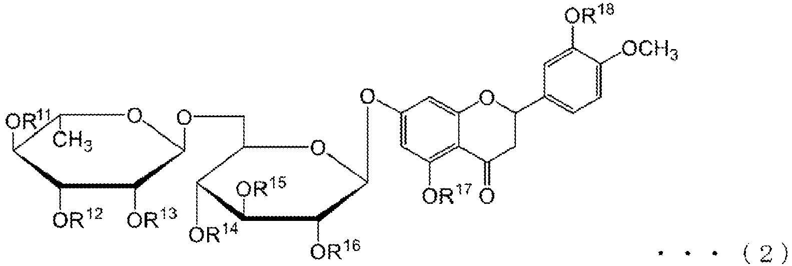 [In Formula (1), R¹ to R⁹ are each independently a methyl group or a hydrogen atom, provided that at least one of R¹ to R⁹ is a methyl group, and
in Formula (2), R¹¹ to R¹⁸ are each independently a methyl group or a hydrogen atom, provided that at least one of R¹¹ to R¹⁸ is a methyl group.]
[3] The barrier function improver according to [2], in which the methyl hesperidin is one or more selected from the group consisting of chalcone form methyl hesperidin represented by General Formula (3) and flavanone form methyl hesperidin represented by General Formula (4). 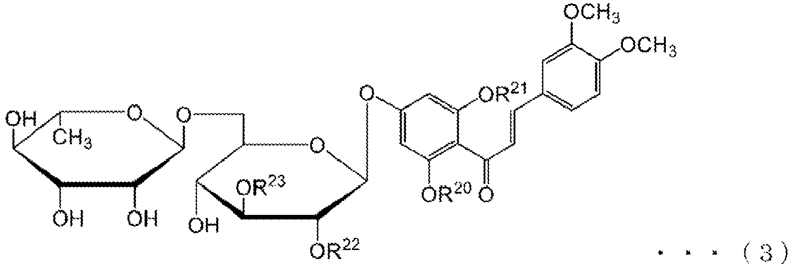 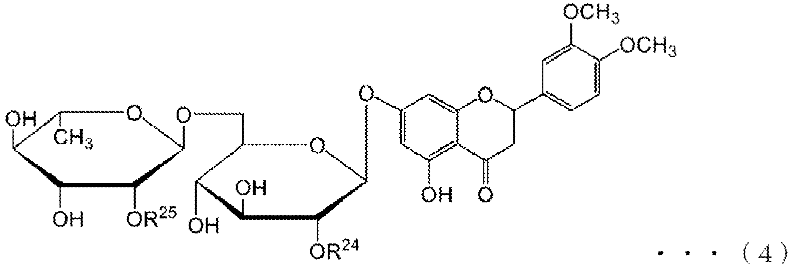 [In Formula (3), R²⁰ to R²³ are each independently a methyl group or a hydrogen atom, and
in Formula (4), R²⁴ and R²⁵ are each independently a methyl group or a hydrogen atom.]
[4] The barrier function improver according to [3], in which the chalcone form methyl hesperidin represented by General Formula (3) is one or more selected from the group consisting of chalcone forms 1 to 3 having a combination of R²⁰ to R²³ shown in Table 1 below.

**[Table 1]**

| | R²⁰ | R²¹ | R²² | R²³ |
|---|---|---|---|---|
| Chalcone form 1 | CH₃ | CH₃ | CH₃ | CH₃ |
| Chalcone form 2 | H | CH₃ | CH₃ | H |
| Chalcone form 3 | H | CH₃ | H | H |

[5] The barrier function improver according to [3] or [4], in which the flavanone form methyl hesperidin represented by General Formula (4) is one or more selected from the group consisting of flavanone forms 1 to 4 having a combination of R²⁴ and R²⁵ shown in Table 2 below.

**[Table 2]**

| | R²⁴ | R²⁵ |
|---|---|---|
| Flavanone form 1 | CH₃ | CH₃ |
| Flavanone form 2 | CH₃ | H |
| Flavanone form 3 | H | H |
| Flavanone form 4 | H | CH₃ |

[6] The barrier function improver according to any one of [1] to [5], in which the barrier function improver is for promoting production of filaggrin.
[7] The barrier function improver according to any one of [1] to [5], in which the barrier function improver is for promoting production of involucrin.
[8] The barrier function improver according to any one of [1] to [5], in which the barrier function improver is for promoting production of loricrin.
[9] The barrier function improver according to any one of [1] to [5], in which the barrier function improver is for promoting production of transglutaminase 1.
[10] The barrier function improver according to any one of [1] to [5], in which the barrier function improver is for promoting production of aquaporin 3.
[11] The barrier function improver according to any one of [1] to [5], in which the barrier function improver is for promoting production of claudin 1.
[12] A barrier-function-improving composition containing: the barrier function improver according to any one of [1] to [11]; and a pharmaceutically acceptable carrier.
[13] The barrier-function-improving composition according to [12], in which a total content of the methyl hesperidin is 0.01% to 2% by mass.

### [Advantageous Effects of Invention]

According to the present invention, a barrier function improver capable of effectively improving a barrier function, and a barrier-function-improving composition containing the above-mentioned barrier function improver can be provided.

### [Brief Description of Drawings]

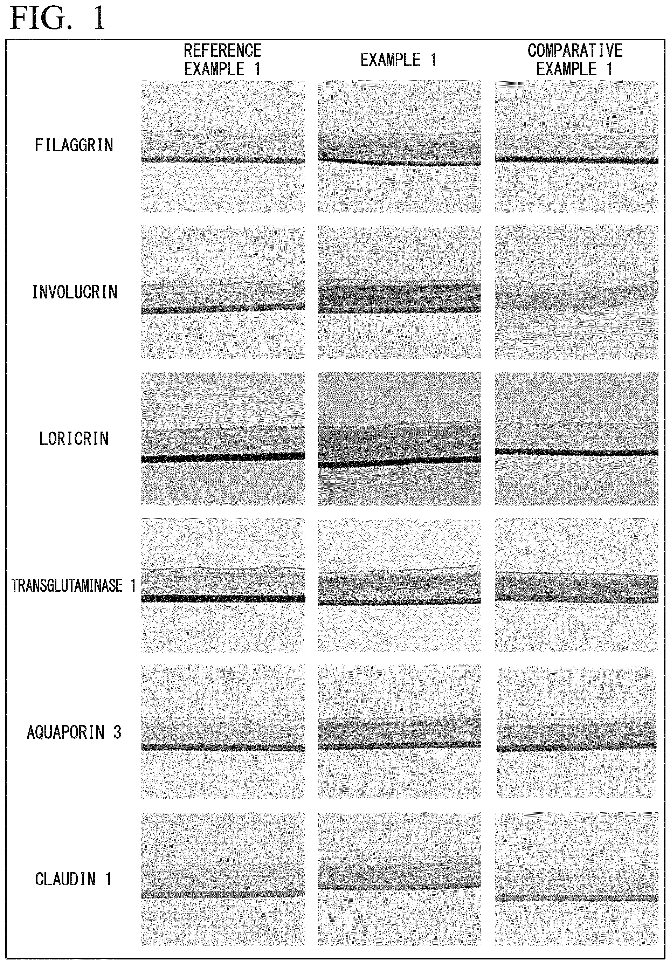

FIG. 1 is a photograph of skin tissue subjected to immune antibody staining of Test Example 1.

### [Description of Embodiments]

### (Barrier function improver)

In one embodiment, the present invention provides a barrier function improver for skin containing methyl hesperidin as an active ingredient.

The term "barrier function" refers to a function of the skin for preventing the evaporation of water inside the skin due to dryness, ultraviolet rays, and other physical and chemical stimuli from the outside, and defending against the invasion of contaminants from the outside.

The barrier function improver according to the present embodiment can effectively improve the barrier function by promoting the production of filaggrin, involucrin, loricrin, and transglutaminase 1, which are differentiation markers, aquaporin 3, which is a water channel of the cell membrane, and claudin 1, which is a tight junction structure.

### <Methyl hesperidin>

The barrier function improver of the present embodiment is not particularly limited as long as it contains methyl hesperidin as an active ingredient. It is preferable that, in the methyl hesperidin used in the barrier function improver of the present embodiment, hesperidin be methylated and solubilized in water.

As the methyl hesperidin, a chalcone-type compound represented by General Formula (1) (chalcone form methyl hesperidin) and a flavanone-type compound represented by General Formula (2) (flavanone form methyl hesperidin) are mainly known.

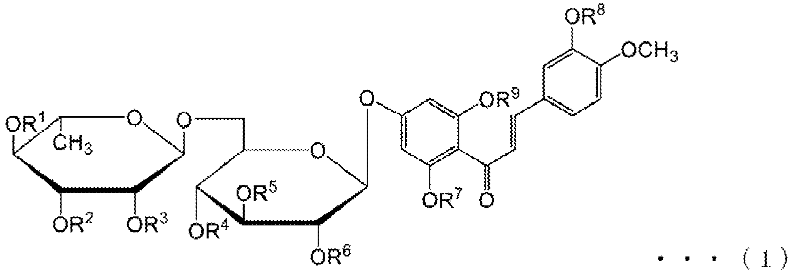

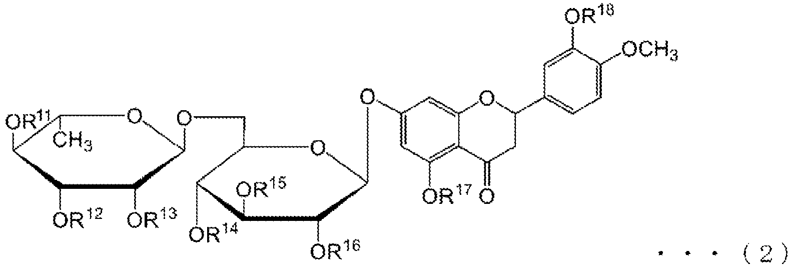

[In Formula (1), R¹ and R⁹ are each independently a methyl group or a hydrogen atom, provided that at least one of R¹ to R⁹ is a methyl group, and
in Formula (2), R¹¹ to R¹⁸ are each independently a methyl group or a hydrogen atom, provided that at least one of R¹¹ to R¹⁸ is a methyl group.]

The methyl hesperidin used in the barrier function improver of the present embodiment is preferably one or more selected from the group consisting of chalcone form methyl hesperidin represented by General Formula (1) and flavanone form methyl hesperidin represented by General Formula (2).

In General Formula (1), R¹ to R⁹ are each independently a methyl group or a hydrogen atom, provided that at least one of R¹ to R⁹ is a methyl group. Among R¹ to R⁹, it is preferable that any one to six be methyl groups, and it is more preferable that any two to five be methyl groups.

In General Formula (2), R¹¹ to R¹⁸ are each independently a methyl group or a hydrogen atom, provided that at least one of R¹¹ to R¹⁸ is a methyl group. Among R¹¹ to R¹⁸, it is preferable that any one to four be methyl groups, and it is more preferable that any one to three be methyl groups.

Among the compounds represented by General Formula (1), the chalcone form methyl hesperidin is preferably a compound represented by General Formula (3) below. Among the compounds represented by General Formula (2), the flavanone form methyl hesperidin is preferably a compound represented by General Formula (4) below.

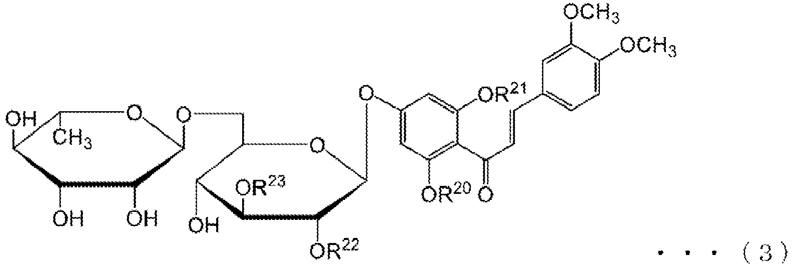

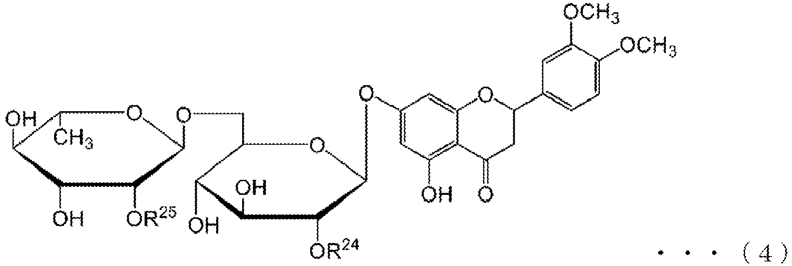

[In Formula (3), R²⁰ to R²³ are each independently a methyl group or a hydrogen atom, and
in Formula (4), R²⁴ and R²⁵ are each independently a methyl group or a hydrogen atom.]

In General Formula (3), R²⁰ to R²³ are each independently a methyl group or a hydrogen atom. The chalcone form methyl hesperidin represented by General Formula (3) is preferably one or more selected from the group consisting of chalcone forms 1 to 3 having a combination of R²⁰ to R²³ shown in Table 3 below.

**[Table 3]**

| | R²⁰ | R²¹ | R²² | R²³ |
|---|---|---|---|---|
| Chalcone form 1 | CH₃ | CH₃ | CH₃ | CH₃ |
| Chalcone form 2 | H | CH₃ | CH₃ | H |
| Chalcone form 3 | H | CH₃ | H | H |

In General Formula (4), R²⁴ and R²⁵ are each independently a methyl group or a hydrogen atom. The flavanone form methyl hesperidin represented by General Formula (4) is preferably one or more selected from the group consisting of flavanone forms 1 to 4 having a combination of R²⁴ and R²⁵ shown in Table 4 below.

**[Table 4]**

| | R²⁴ | R²⁵ |
|---|---|---|
| Flavanone form 1 | CH₃ | CH₃ |
| Flavanone form 2 | CH₃ | H |
| Flavanone form 3 | H | H |
| Flavanone form 4 | H | CH₃ |

The methyl hesperidin used in the barrier function improver of the present embodiment may be one type alone or may be a mixture of two or more types. The methyl hesperidin may include both of or only one of the chalcone form methyl hesperidin represented by General Formula (1) or the chalcone form methyl hesperidin by General Formula (3), and the flavanone form methyl hesperidin represented by General Formula (2) or the flavanone form methyl hesperidin represented by General Formula (4). The methyl hesperidin may include the chalcone form methyl hesperidin represented by General Formula (3) and the flavanone form methyl hesperidin represented by General Formula (4). In addition, the methyl hesperidin may include any one or more of the above-mentioned chalcone forms 1 to 3, or may include any one or more of the above-mentioned flavanone forms 1 to 4.

In addition, the barrier function improver of the present embodiment may contain a mixture of the chalcone forms 1 to 3 and the flavanone forms 1 to 3 as the methyl hesperidin.

The methyl hesperidin can be produced by a known method. Examples of the known method include a production method of dissolving hesperidin produced from the peel of citrus fruits or the like in an aqueous sodium hydroxide solution, reacting the alkaline solution thereof with a corresponding amount of dimethyl sulfate to neutralize the reaction liquid with sulfuric acid, extracting with n-butyl alcohol to evaporate the solvent, and thereafter recrystallizing with isopropyl alcohol (Sakieki, Nippon kagaku zassi, (1958) Vol. 79, pp. 733-736; Japanese Patent No. 6312333), but the production method is not limited thereto.

It is also possible to purchase and use a commercially available product (for example, those distributed as pharmaceutical additives, food additives, and cosmetic raw materials, or "Methyl Hesperidin" (Showa Denko K.K.), "Methyl Hesperidin" (Tokyo Chemical Industry Co., Ltd.), "Hesperidin methyl chalcone" (Sigma-Aldrich), and the like).

The barrier function improver of the present embodiment can be used by administering itself to a patient for the purpose of treating rough skin, dry skin, psoriasis, atopic dermatitis, and the like. The barrier function improver of the present embodiment can also be used by being blended in pharmaceuticals or cosmetics for the purpose of improving the barrier function. The barrier function improver of the present embodiment may also be used by being blended in a barrier-function-improving composition to be described later.

The barrier function improver of the present embodiment can effectively improve the barrier function by promoting the production of filaggrin.

The barrier function improver of the present embodiment can effectively improve the barrier function by promoting the production of involucrin.

The barrier function improver of the present embodiment can effectively improve the barrier function by promoting the production of loricrin.

The barrier function improver of the present embodiment can effectively improve the barrier function by promoting the production of transglutaminase 1.

The barrier function improver of the present embodiment can effectively improve the barrier function by promoting the production of aquaporin 3.

The barrier function improver of the present embodiment can effectively improve the barrier function by promoting the production of claudin 1.

The barrier function improver of the present embodiment can effectively improve the barrier function, and thus can be used for preventing or treating rough skin, dry skin, psoriasis, and atopic dermatitis.

### (Filaggrin)

Filaggrin is a kind of basic protein produced by granular cells in the epidermis and acts to promote the fiber formation reaction of keratin fibers progressing in cells of the stratum corneum. In the process of translocation of cells of the stratum corneum from a lower layer to an upper layer, filaggrin is further decomposed into amino acids by the action of proteases, and free amino acids in the stratum corneum function as natural moisturizing factors (NMFs). When the production of filaggrin is promoted, the amount of the NMFs is increased and the moisturizing power of the stratum corneum is enhanced, thereby improving the barrier function of the skin.

### (Involucrin)

Involucrin is one of major proteins constituting the cornified envelope of cells of the stratum corneum, and is used as an index of the early stage of differentiation of keratinocytes (epidermal keratinocytes). When the production of involucrin is promoted, the formation of a cornified envelope, which is a structure that has a moisturizing power of the stratum corneum, is promoted, thereby improving the barrier function of the skin.

### (Loricrin)

Loricrin is a hydrophobic protein containing a large amount of glycine, serine, cysteine, and the like, and is a constituent component of a cornified envelope. When the production of involucrin is promoted, the formation of cornified envelopes is promoted, thereby improving the barrier function of the skin.

### (Transglutaminase 1)

Transglutaminase 1 is an enzyme that forms an isopeptide bond between glutamine and lysine in the protein, and is involved in the formation reaction of a cornified envelope. When the production of transglutaminase 1 is promoted, the formation of cornified envelopes is promoted, thereby improving the barrier function of the skin.

### (Aquaporin 3)

Aquaporin 3 is a protein having pores present in the cell membrane, and functions as a water channel that regulates the water amount in cells. When the production of aquaporin 3 is promoted, the water amount in epidermal cells is increased, thereby improving the barrier function of the epidermis.

### (Claudin 1)

Claudin 1 is a major protein at tight junctions in cell-cell junctions and constructs an intercellular barrier. When the production of claudin 1 is promoted, the formation of tight junctions is promoted, thereby improving the barrier function of the epidermis.

The barrier function improver of the present embodiment may be used to prevent rough skin, dry skin, psoriasis, atopic dermatitis, or the like by being administered to a patient at high risk of developing rough skin, dry skin, psoriasis, atopic dermatitis, or the like. The barrier function improver of the present embodiment may also be used to inhibit the progression or exacerbation of rough skin, dry skin, psoriasis, atopic dermatitis, or the like by being administered to a patient who has developed rough skin, dry skin, psoriasis, atopic dermatitis, or the like.

The barrier function improver of the present embodiment can be administered to a patient in the same manner as the barrier-function-improving composition to be described later, and may be administered orally or parenterally, may be administered intravenously, intraarterially, intramuscularly, intracutaneously, subcutaneously, intraperitoneally, or the like, may be administered rectally as a suppository, or may be administered to the skin as an external preparation for skin.

### (Barrier-function-improving composition)

The barrier-function-improving composition of the present embodiment contains the above-mentioned barrier function improver containing methyl hesperidin, and a pharmaceutically acceptable carrier.

The barrier-function-improving composition of the present embodiment can be produced by mixing the above-mentioned barrier function improver, a pharmaceutically acceptable carrier, and optionally other components, and formulating them according to a general method (for example, a method described in the Japanese Pharmacopoeia).

In the present specification, the "pharmaceutically acceptable carrier" means a carrier that does not inhibit a physiological activity of an active ingredient and does not exhibit substantial toxicity with respect to its administration target.

The phrase "does not exhibit substantial toxicity" means that this component does not exhibit toxicity with respect to an administration target in a generally used dosage.

The pharmaceutically acceptable carrier is not particularly limited, and examples thereof include excipients, binders, disintegrants, lubricants, stabilizers, diluents, solvents for injections, moisturizers, touch sensation improvers, surfactants, polymer-thickening-gelling agents, solvents, propellants, antioxidants, reducing agents, oxidizing agents, chelating agents, acids, alkali, powders, inorganic salts, water, metal-containing compounds, unsaturated monomers, polyhydric alcohols, polymer additives, wetting agents, thickeners, tackifiers, oily raw materials, liquid matrices, fat-soluble substances, and polymer carboxylate salts.

Specific examples of these components include those described in PCT International Publication No. WO2016/076310. In addition, specific examples of the polymer-thickening-gelling agents include methacryloyloxyethyl phosphorylcholine, butyl methacrylate, and polymers thereof.

For the pharmaceutically acceptable carrier in the barrier-function-improving composition of the present embodiment, one type may be used alone, or two or more types may be used in combination.

In addition, the other components are not particularly limited, but examples thereof include preservatives, antibacterial agents, ultraviolet absorbents, whitening agents, vitamins and derivatives thereof other than methyl hesperidin, antiphlogistics, anti-inflammatory agents, hair growth drugs, blood circulation promoters, stimulants, hormones, anti-wrinkle agents, anti-aging agents, tightening agents, cooling sensation agents, warming sensation agents, wound healing promoters, irritation relieving agents, analgesics, cell-activating agents, plant/animal/microbial extracts, seed oils, antipruritic agents, keratin exfoliating and dissolving agents, antiperspirants, refreshing agents, astringents, enzymes, nucleic acids, fragrances, colorants, coloring agents, dyes, pigments, anti-inflammatory analgesics, antifungals, antihistamines, hypnotic sedatives, tranquilizers, antihypertensives, antihypertensive diuretics, antibiotics, anesthetics, antibacterial substances, antiepileptic agents, coronary vasodilators, herbal medicines, anti-itch drugs, keratin softening and exfoliating agents, ultraviolet blockers, disinfectants, antioxidant substances, pH adjusters, additives, and metal soaps. Specific examples of these components include those described in PCT International Publication No. WO2016/076310. In addition, specific examples of the plant/animal/microbial extracts include Lapsana communis flowers/leaves/stems, and Camellia sinensis leaves. Specific examples of the seed oils include a Moringa oleifera seed oil. Specific examples of the fragrances include perillaldehyde.

For the other component, one type may be used alone, or two or more types may be used in combination.

The barrier-function-improving composition of the present embodiment can contain the above-mentioned barrier function improver in a therapeutically effective amount. The term "therapeutically effective amount" means the amount of a drug effective for treating or preventing diseases of patients. The therapeutically effective amount may vary depending on the disease state, age, gender, body weight, and the like of an administration target.

In barrier-function-improving composition of the present embodiment, the therapeutically effective amount of the above-mentioned barrier function improver may be an amount in which methyl hesperidin can improve the barrier function. In addition, the therapeutically effective amount of the above-mentioned barrier function improver may be an amount in which methyl hesperidin can promote the production of at least one selected from the group consisting of filaggrin, involucrin, loricrin, transglutaminase 1, aquaporin 3, and claudin 1.

The therapeutically effective amount (total content of methyl hesperidin) of the above-mentioned barrier function improver in the barrier-function-improving composition of the present embodiment may be 0.01% to 2% by mass for example, may be 0.05% to 1.5% by mass for example, or may be 0.1% to 1.0% by mass for example, with respect to the total amount of the barrier-function-improving composition.

The term the "total content of methyl hesperidin" means, in a case where one type of methyl hesperidin is used alone, the content of the compound, and means, in a case where two or more types of methyl hesperidin are used in combination, the total content of these compounds.

Preferable specific examples of the barrier-function-improving composition of the present embodiment include those having the following composition.

(Example 1) A barrier-function-improving composition containing: 0.01% to 2% by mass of methyl hesperidin; 0.04% to 1% by mass of dipotassium glycyrrhizinate; and 0.01% to 10% by mass of sodium hyaluronate.

(Example 2) A barrier-function-improving composition containing: 0.01% to 2% by mass of methyl hesperidin; 0.05% to 10% by mass of isononyl isononanoate; 0.05% to 10% by mass of octyldodecyl myristate; and 0.001 % to 5% by mass of tocopherol.

The barrier-function-improving composition of the present embodiment may be a pharmaceutical composition or a cosmetic preparation.

### (Pharmaceutical composition)

In one embodiment, the present invention provides a barrier-function-improving pharmaceutical composition containing the above-mentioned barrier function improver and a pharmaceutically acceptable carrier.

In the pharmaceutical composition of the present embodiment, the pharmaceutically acceptable carrier is not particularly limited, and carriers generally used for pharmaceuticals can be used in addition to the carriers in the above-mentioned examples. For example, it is possible to use general raw materials described in the Japanese Pharmacopoeia, the Japanese Pharmaceutical Codex, Japanese Pharmaceutical Excipients 2013 (Yakuji Nippo, Ltd., 2013), the Japanese Pharmaceutical Excipients Directory 2016 (edited by International Pharmaceutical Excipients Council Japan, Yakuji Nippo, Ltd., 2016), Handbook of Pharmaceutical Excipients, 7th edition (Pharmaceutical Press, 2012), and the like.

For the pharmaceutically acceptable carrier, one type may be used alone, or two or more types may be used in combination.

The pharmaceutical composition of the present embodiment may contain other components in addition to the above-mentioned barrier function improver and the pharmaceutically acceptable carriers. The other components are not particularly limited, and general pharmaceutical additives can be used. Furthermore, as the other components, it is also possible to use an active ingredient other than the above-mentioned barrier function improver. As the pharmaceutical additives and the active ingredients as the other components, in addition to the other components in the above-mentioned examples, it is possible to use general raw materials described in, for example, the Japanese Pharmacopoeia, the Japanese Pharmaceutical Codex, Japanese Pharmaceutical Excipients 2013 (Yakuji Nippo, Ltd., 2013), the Japanese Pharmaceutical Excipients Directory 2016 (edited by International Pharmaceutical Excipients Council Japan, Yakuji Nippo, Ltd., 2016), Handbook of Pharmaceutical Excipients, 7th edition (Pharmaceutical Press, 2012), and the like. For the other component, one type may be used alone, or two or more types may be used in combination.

A dosage form of the pharmaceutical composition of the present embodiment is not particularly limited, and can be set to a dosage form generally used for pharmaceutical preparations. Examples thereof include orally administered dosage forms such as tablets, coated tablets, pills, powders, granules, capsules, solutions, suspensions, and emulsions; and parenterally administered dosage forms such as injections, suppositories, and external preparations for skin. The pharmaceutical composition in these dosage forms can be formulated according to a general method (for example, a method described in the Japanese Pharmacopoeia).

A method for administering the pharmaceutical composition of the present embodiment is not particularly limited, and administration can be performed by a method generally used as a method for administering pharmaceuticals. For example, administration may be performed orally as tablets, coated tablets, pills, powders, granules, capsules, solutions, suspensions, emulsions, and the like; administration maybe performed intravenously, intraarterially, intramuscularly, intracutaneously, subcutaneously, intraperitoneally, and the like as injections, infusion preparations, and the like alone, or as a mixture with common infusions such as a glucose solution and a Ringer's solution; administration may be performed rectally as suppositories; or administration may be performed to skin as external preparations for skin.

The dosage of the pharmaceutical composition of the present embodiment can be set to a therapeutically effective amount. The therapeutically effective amount may be appropriately determined according to the symptoms, body weight, age, gender, and the like of a patient, a dosage form of the pharmaceutical composition, an administration method, and the like.

For example, the dosage of the pharmaceutical composition of the present embodiment is 0.01 to 500 mg per dosage unit form as the total content of methyl hesperidin in a case of oral administration, is 0.02 to 250 mg per dosage unit form as the total content of methyl hesperidin in a case of injections, is 0.01 to 500 mg per dosage unit form as the total content of methyl hesperidin in a case of suppositories, and is 0.01 to 500 mg per dosage unit form as the total content of methyl hesperidin in a case of external preparations for skin.

The administration interval of the pharmaceutical composition of the present embodiment may be appropriately determined according to the symptoms, body weight, age, gender, and the like of a patient, a dosage form of the pharmaceutical composition, an administration method, and the like. For example, the administration interval may be set to once a day, about 2 to 3 times a day, or the like.

The pharmaceutical composition of the present embodiment can be used for treating or preventing symptoms or diseases caused by a deterioration of the barrier function. Examples of such symptoms or diseases include rough skin, dry skin, psoriasis, and atopic dermatitis.

In addition, the pharmaceutical composition of the present embodiment can be used to inhibit the progression of rough skin, dry skin, psoriasis, or atopic dermatitis by being administered to a patient with rough skin, dry skin, psoriasis, or atopic dermatitis, for example. In addition, the pharmaceutical composition of the present embodiment can be used to treat rough skin, dry skin, psoriasis, or atopic dermatitis by being administered to a patient with rough skin, dry skin, psoriasis, or atopic dermatitis. In addition, the pharmaceutical composition of the present embodiment can be used to treat symptoms or diseases caused by decreased production of filaggrin, involucrin, loricrin, transglutaminase 1, aquaporin 3, or claudin 1.

The pharmaceutical composition of the present embodiment can also be used to prevent rough skin, dry skin, psoriasis, or atopic dermatitis by being administered to a patient at high risk of developing rough skin, dry skin, psoriasis, or atopic dermatitis.

### (Cosmetic preparation)

In one embodiment, the present invention provides a cosmetic preparation for improving the barrier function, the cosmetic preparation containing the above-mentioned barrier function improver and a pharmaceutically acceptable carrier.

In the cosmetic preparation of the present embodiment, the pharmaceutically acceptable carrier is not particularly limited, and carriers generally used for cosmetic preparations can be used in addition to the carriers in the above-mentioned examples. For example, it is possible to use general raw materials described in the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like.

For the pharmaceutically acceptable carrier, one type may be used alone, or two or more types may be used in combination.

The cosmetic preparation of the present embodiment may contain other components in addition to the above-mentioned barrier function improver and the pharmaceutically acceptable carriers. The other components are not particularly limited, and general additives for cosmetics can be used. Furthermore, as the other components, it is also possible to use an active ingredient other than the above-mentioned barrier function improver. As the additives for cosmetics and the active ingredients as the other components, in addition to the other components in the above-mentioned examples, it is possible to use general raw materials described in, for example, the Japanese Standards of Cosmetic Ingredients, Second Edition, Supplements (edited by the Pharmaceutical and Medical Device Regulatory Science Society of Japan, Yakuji Nippo, Ltd., 1984); the Japanese Cosmetic Ingredients Codex (supervisorily edited by the Examination Division of the Pharmaceutical Affairs Bureau, Yakuji Nippo, Ltd., 1993); Supplement to the Japanese Cosmetic Ingredients Codex (supervisorily edited by the Examination Division of the Pharmaceutical Affairs Bureau, Yakuji Nippo, Ltd., 1993); the Comprehensive Licensing Standards of Cosmetics by Category (supervisorily edited by the Examination Division of the Pharmaceutical Affairs Bureau, Yakuji Nippo, Ltd., 1993); the Dictionary of Cosmetic Ingredients (Nikko Chemicals Co., Ltd., 1991); International Cosmetic Ingredient Dictionary and Handbook 2002, Ninth Edition, Vol. 1 to 4, CTFA; and the like. For the other component, one type may be used alone, or two or more types may be used in combination.

The form of the cosmetic preparation of the present embodiment is not particularly limited, and can be set to a form generally used for cosmetic preparations. Examples thereof include hair cosmetic preparations such as shampoos, hair conditioners, and hairdressing agents; basic cosmetic preparations such as facial cleansers, cleansing agents, skin toners, emulsions, lotions, creams, gels, sunscreens, packs, masks, and serums; makeup cosmetic preparations such as foundations, makeup primers, lipsticks, lip glosses, and blushers; body cosmetic preparations such as body cleansers, body powders, and deodorant cosmetics; and the like. These cosmetic preparations can be produced according to a general method.

In addition, a dosage form of the cosmetic preparation of the present embodiment is not particularly limited, but examples thereof include emulsified types such an oil-in-water (O/W) type, a water-in-oil (W/O) type, a W/O/W type, and an O/W/O type, emulsified polymer types, oily types, solid types, liquid types, kneaded types, stick types, volatile oil types, powder types, jelly types, gel types, paste types, cream types, sheet types, film types, mist types, spray types, aerosol types, multilayer types, foam types, flake types, and the like.

The use amount of the cosmetic preparation of the present embodiment is not particularly limited, but can be set to an amount effective for improving the barrier function.

For example, the use amount of the cosmetic preparation of the present embodiment is 0.01 to 500 mg per one time use as the total content of methyl hesperidin, and may be 0.15 to 300 mg for example, may be 0.15 to 200 mg for example, or may be 0.2 to 100 mg for example.

The use interval of the cosmetic preparation of the present embodiment is not particularly limited, but can be set to once a day, about 2 to 3 times a day, or the like, for example.

The cosmetic preparation of the present embodiment can be used to alleviate symptoms caused by a deterioration of the barrier function. Alternatively, to prevent the development of symptoms caused by a deterioration of the barrier function, the cosmetic preparation of the present embodiment may be used for daily skin care and make-up by subjects having a high risk of developing these symptoms.

### (Other embodiments)

In one embodiment, the present invention provides a method for improving a barrier function, the method including a step of administering methyl hesperidin to a target.

In one embodiment, the present invention provides a method for promoting the production of filaggrin, involucrin, loricrin, or transglutaminase 1, the method including a step of administering methyl hesperidin to a target.

In one embodiment, the present invention provides a method for promoting the production of aquaporin 3, the method including a step of administering methyl hesperidin to a target.

In one embodiment, the present invention provides a method for promoting the production of claudin 1, the method including a step of administering methyl hesperidin to a target.

In one embodiment, the present invention provides methyl hesperidin for improving a barrier function.

In one embodiment, the present invention provides methyl hesperidin for promoting the production of filaggrin, involucrin, loricrin, or transglutaminase 1.

In one embodiment, the present invention provides methyl hesperidin for promoting the production of aquaporin 3.

In one embodiment, the present invention provides methyl hesperidin for promoting the production of claudin 1.

In one embodiment, the present invention provides methyl hesperidin for preventing or treating rough skin, dry skin, psoriasis, or atopic dermatitis.

In one embodiment, the present invention provides use of methyl hesperidin for producing the barrier function improver.

In one embodiment, the present invention provides use of methyl hesperidin for producing a production promoter of filaggrin, involucrin, loricrin, or transglutaminase 1.

In one embodiment, the present invention provides use of methyl hesperidin for producing a production promoter of aquaporin 3.

In one embodiment, the present invention provides use of methyl hesperidin for producing a production promoter of claudin 1.

In one embodiment, the present invention provides use of methyl hesperidin for producing the barrier-function-improving composition.

In one embodiment, the present invention provides use of methyl hesperidin for producing a production-promoting composition of filaggrin, involucrin, loricrin, or transglutaminase 1.

In one embodiment, the present invention provides use of methyl hesperidin for producing a production-promoting composition of aquaporin 3.

In one embodiment, the present invention provides use of methyl hesperidin for producing a production-promoting composition of claudin 1.

### [Examples]

The present invention will be described in more detail below with reference to examples, but the present invention is not limited to these examples.

### [Methyl hesperidin]

In the following test examples, methyl hesperidin (product name: Methyl Hesperidin) manufactured by Showa Denko K.K. was used. This product is a mixture in which the total content of the above-mentioned chalcone forms 1 to 3 and the above-mentioned flavanone forms 1 to 3 is 97.5% by mass or more in the total amount of the composition. In Test Example 1 and Test Example 2, a "methyl hesperidin DMSO aqueous solution" in which methyl hesperidin was dissolved in a DMSO aqueous solution was used.

### [Hesperidin]

In the following test examples, hesperidin manufactured by Tokyo Chemical Industry Co., Ltd. was used. In Test Example 1 and Test Example 2, a "hesperidin DMSO aqueous solution" in which hesperidin was dissolved in a DMSO aqueous solution was used.

### [Test Example 1] Effect of promoting expression of differentiation marker, aquaporin 3, and claudin 1

By the following method, the effect of promoting the expression of differentiation markers (filaggrin, involucrin, loricrin, transglutaminase 1), aquaporin 3, and claudin 1 in 3D human model skin was verified.

The 3D human model skin (manufactured by KURABO INDUSTRIES LTD.) was transplanted into an EPI-100 medium (manufactured by KURABO INDUSTRIES LTD.) and cultured for 30 minutes. Subsequently, in Example 1, 30 µL of a methyl hesperidin DMSO aqueous solution in which 0.2% (V/V) methyl hesperidin was dissolved in a 0.1% (V/V) DMSO aqueous solution was added to the surface of the skin, and cultured for 6 hours. In Comparative Example 1, 30 µL of a hesperidin DMSO aqueous solution in which 0.2% (V/V) hesperidin was dissolved in a 0.1% (V/V) DMSO aqueous solution was added to the surface of the skin, and cultured for 6 hours. In Reference Example 1, a 0.1% (V/V) DMSO aqueous solution was added to the surface of the skin and cultured for 6 hours. Thereafter, the skin was washed with physiological saline, fixed with ALTFiX (registered trademark) (manufactured by FALMA Corporation), and thereafter embedded in paraffin to produce a skin tissue section.

The section was deparaffinized and dewatered. Thereafter, immune antibody staining of the skin tissue was performed. As primary antibodies, filaggrin, involucrin, loricrin, aquaporin 3, and claudin 1 (all manufactured by Abcam plc.), and transglutaminase 1 (manufactured by Novus Biologicals) were used. Each antibody was diluted using a 0.05% twin-20-containing phosphate buffer solution at a dilution ratio as follows: filaggrin (1:200), involucrin (1:150), loricrin (1:150), transglutaminase 1 (1:500), aquaporin 3 (1:500), and claudin 1 (1:100). As a secondary antibody, a peroxidase-binding anti-rabbit IgG antibody (manufactured by Abcam plc.) derived from western wasabi was diluted with a 0.05% twin-20-containing phosphate buffer solution at 1:200 and used. After washing after the antibody reaction, a coloring reaction was carried out using a peroxidase chromogenic substrate, 3,3'-diaminobenzidine tetrahydrochloride (manufactured by FUJIFILM Wako Pure Chemical Corporation).

Each tissue was observed with a Nikon TS1, and a photograph was taken. For each of the three independent images obtained, quantitative analysis of a brightness value correlating with a protein expression level was performed using ImageJ software (NIH). Each analysis result of Example 1 and Comparative Example 1 is shown as a relative value when an average analysis value of Reference Example 1 was set to 1.00. FIG. 1 shows complete response examples of the captured photographs, and Table 5 shows the results of the quantitative analysis.

**[Table 5]**

| | Reference Example 1 | Example 1 | Comparative Example 1 |
|---|---|---|---|
| | DMSO aqueous solution | Methyl hesperidin DMSO aqueous solution | Hesperidin DMSO aqueous solution |
| Filaggrin | 1.00 | 2.52 | 1.24 |
| Involucrin | 1.00 | 2.34 | 1.04 |
| Loricrin | 1.00 | 2.60 | 0.70 |
| Transglutaminase 1 | 1.00 | 1.39 | 0.78 |
| Aquaporin 3 | 1.00 | 1.20 | 0.76 |
| Claudin 1 | 1.00 | 2.67 | 0.61 |

From the results shown in FIG. 1 and Table 5, in Example 1 to which the methyl hesperidin DMSO aqueous solution was added, the effect of promoting the expression was recognized in all of filaggrin, involucrin, loricrin, and transglutaminase 1, which are differentiation markers, aquaporin 3, and claudin 1 which is a tight junction structure, as compared to Reference Example 1 to which the DMSO aqueous solution was added and Comparative Example 1 to which the hesperidin DMSO aqueous solution was added.

### [Test Example 2] Effect of improving transepidermal water loss (TEWL) of skin

The effect of improving transepidermal water loss (TEWL) in 3D human model skin was verified by the following method.

The 3D human model skin (manufactured by KURABO INDUSTRIES LTD.) was transplanted into an EPI-100 medium (manufactured by KURABO INDUSTRIES LTD.) and cultured for 30 minutes. Subsequently, in Example 2, 30 µL of a methyl hesperidin DMSO aqueous solution in which 0.2% (V/V) methyl hesperidin was dissolved in a 0.1% (V/V) DMSO aqueous solution was added to the surface of the skin, and cultured for 6 hours. In Comparative Example 2, 30 µL of a hesperidin DMSO aqueous solution in which 0.2% (V/V) hesperidin was dissolved in a 0.1% (V/V) DMSO aqueous solution was added to the surface of the skin, and cultured for 6 hours. In Reference Example 2, a 0.1% (V/V) DMSO aqueous solution was added to the surface of the skin and cultured for 6 hours. Thereafter, the skin was washed with a phosphate buffer solution and further cultured. After the addition of each of the above-mentioned samples, a transepidermal water loss (TEWL) at 72 hours was measured with VAPO SCAN (manufactured by ASCH JAPAN Co., Ltd.). The measurement values of Example 2 and Comparative Example 2 were shown as relative values when the measurement value of Reference Example 2 was set to 1.00. Table 6 shows the results.

**[Table 6]**

| | Reference Example 2 | Example 2 | Comparative Example 2 |
|---|---|---|---|
| | DMSO aqueous solution | Methyl hesperidin DMSO aqueous solution | Hesperidin DMSO aqueous solution |
| Transepidermal water loss (TEWL) | 1.00 | 0.80 | 0.93 |

From the results shown in Table 6, in Example 2 to which the methyl hesperidin DMSO aqueous solution was added, it was confirmed that the transepidermal water loss (TEWL) decreased and that the barrier function was enhanced, as compared to Reference Example 2 to which the DMSO aqueous solution was added and Comparative Example 2 to which the hesperidin DMSO aqueous solution was added.

### [Test Example 3] Sensory evaluation by use test of barrier-function-improving composition

Barrier-function-improving compositions shown in Tables 8 to 10 were produced. Table 8 (Comparative Examples 3 to 5 and Examples 3 and 4) shows the prescriptions of skin toners. Table 9 (Comparative Examples 6 to 8 and Examples 5 and 6) shows the prescriptions of creams. Table 10 (Comparative Examples 9 to 11 and Examples 7 and 8) shows the prescriptions of serums.

Samples of each prescription of Examples 3 to 8 and Comparative Examples 3 to 11 were applied to the inside of the lower arm once a day in the evening for three consecutive days for three female targets. Sensory evaluation was performed by determining "dryness", "stiffness", and "itching and tingling sensation" accompanying rough skin at the test site before the start of use and after the completion of use based on the scores shown in Table 7. Abnormalities such as a skin irritation response were not recognized at the test sites of the subjects during the test period and after the test.

The average score value of the sensory evaluation is shown in Tables 8 to 10. The lower the average score value, the better the sensation when use as the barrier-function-improving composition.

**[Table 7]**

| Evaluation criteria | Score |
|---|---|
| Felt much | 5 |
| Felt a little bit | 4 |
| Not changed from before use | 3 |
| Not felt much | 2 |
| Not felt at all | 1 |

**[Table 8]**

| Component (% by mass) | | Comparative Example 3 | Comparative Example 4 | Comparative Example 5 | Example 3 | Example 4 |
|---|---|---|---|---|---|---|
| Methyl hesperidin | | - | - | - | 0.2 | 0.5 |
| Hesperidin | | - | 0.2 | 0.5 | - | - |
| Glycerin | | 4.0 | 4.0 | 4.0 | 4.0 | 4.0 |
| 1,3-Butylene glycol | | 4.0 | 4.0 | 4.0 | 4.0 | 4.0 |
| Potassium citrate | | 5.1 | 5.1 | 5.1 | 5.1 | 5.1 |
| Methylparaben | | 0.2 | 0.2 | 0.2 | 0.2 | 0.2 |
| Sodium hyaluronate (1% aqueous solution) | | 4.0 | 4.0 | 4.0 | 4.0 | 4.0 |
| Dipotassium glycyrrhizinate | | 0.2 | 0.2 | 0.2 | 0.2 | 0.2 |
| Water | | 82.5 | 82.3 | 82.0 | 82.3 | 82.0 |
| Sensory evaluation (Average score) | Dryness | 4.7 | 4.3 | 4.3 | 2.3 | 2.0 |
| | Stiffness | 4.3 | 4.3 | 4.3 | 2.3 | 1.7 |
| | Itching and tingling sensation | 3.7 | 4.3 | 3.7 | 2.7 | 2.0 |

**[Table 9]**

| Component (% by mass) | | Comparative Example 6 | Comparative Example 7 | Comparative Example 8 | Example 5 | Example 6 |
|---|---|---|---|---|---|---|
| Methyl hesperidin | | - | - | - | 0.2 | 0.5 |
| Hesperidin | | - | 0.2 | 0.5 | - | - |
| Hydrogenated rapeseed oil alcohol | | 3.2 | 3.2 | 3.2 | 3.2 | 3.2 |
| Isononyl isononanoate | | 4.8 | 4.8 | 4.8 | 4.8 | 4.8 |
| Squalane | | 9.6 | 9.6 | 9.6 | 9.6 | 9.6 |
| Octyldodecyl myristate | | 4.4 | 4.4 | 4.4 | 4.4 | 4.4 |
| Polyglyceryl-10 isostearate | | 1.5 | 1.5 | 1.5 | 1.5 | 1.5 |
| Glyceryl stearate | | 0.5 | 0.5 | 0.5 | 0.5 | 0.5 |
| Tocopherol | | 0.2 | 0.2 | 0.2 | 0.2 | 0.2 |
| Xanthan gum | | 0.1 | 0.1 | 0.1 | 0.1 | 0.1 |
| Propylparaben | | 0.1 | 0.1 | 0.1 | 0.1 | 0.1 |
| Glycerin | | 4.8 | 4.8 | 4.8 | 4.8 | 4.8 |
| 1,3-Butylene glycol | | 4.0 | 4.0 | 4.0 | 4.0 | 4.0 |
| Methylparaben | | 0.2 | 0.2 | 0.2 | 0.2 | 0.2 |
| Water | | 66.6 | 66.4 | 66.1 | 66.4 | 66.1 |
| Sensory evaluation (Average score) | Dryness | 3.7 | 2.7 | 2.3 | 2.3 | 1.7 |
| | Stiffness | 2.7 | 2.7 | 2.7 | 2.3 | 1.3 |
| | Itching and tingling sensation | 2.7 | 2.3 | 2.3 | 1.7 | 1.7 |

**[Table 10]**

| Component (% by mass) | | Comparative Example 9 | Comparative Example 10 | Comparative Example 11 | Example 7 | Example 8 |
|---|---|---|---|---|---|---|
| Methyl hesperidin | | - | - | - | 0.2 | 0.5 |
| Hesperidin | | - | 0.2 | 0.5 | - | - |
| Glycerin | | 4.0 | 4.0 | 4.0 | 4.0 | 4.0 |
| Potassium citrate | | 1.0 | 1.0 | 1.0 | 1.0 | 1.0 |
| Methylparaben | | 0.2 | 0.2 | 0.2 | 0.2 | 0.2 |
| (PEG-240/decyltetradeceth-20/HDI) copolymer | | 1.5 | 1.5 | 1.5 | 1.5 | 1.5 |
| Water | | 93.3 | 93.1 | 92.8 | 93.1 | 92.8 |
| Sensory evaluation (Average score) | Dryness | 4.7 | 3.3 | 2.7 | 2.7 | 2.0 |
| | Stiffness | 4.0 | 3.7 | 3.3 | 2.3 | 2.0 |
| | Itching and tingling sensation | 4.0 | 3.7 | 3.3 | 2.0 | 1.7 |

From the results shown in Tables 8 to 10, in the prescriptions of Examples 3 to 8, it was confirmed that each average score for dryness, stiffness, and itching and tingling sensation was low and that the barrier-function-improving composition was highly useful, as compared to the prescriptions of Comparative Examples 3 to 11.

### [Industrial Applicability]

According to the present invention, the barrier function improver capable of effectively improving the barrier function, and the barrier-function-improving composition containing the above-mentioned barrier function improver are provided.

Although the preferable examples of the present invention have been described above, the present invention is not limited to these examples. The addition, omission, substitution, and other modifications of the configuration are possible within a range not departing from the spirit of the present invention. The present invention is not limited by the foregoing description, but only by the scope of the appended claims.

## Claims

1. A barrier function improver for skin comprising:
methyl hesperidin as an active ingredient.

2. The barrier function improver according to Claim 1,
wherein the methyl hesperidin is one or more selected from the group consisting of chalcone form methyl hesperidin represented by General Formula (1) and flavanone form methyl hesperidin represented by General Formula (2), 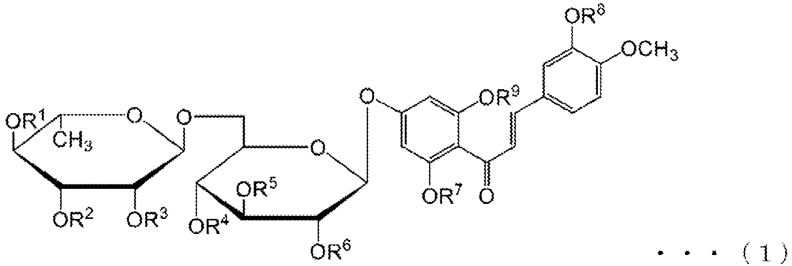 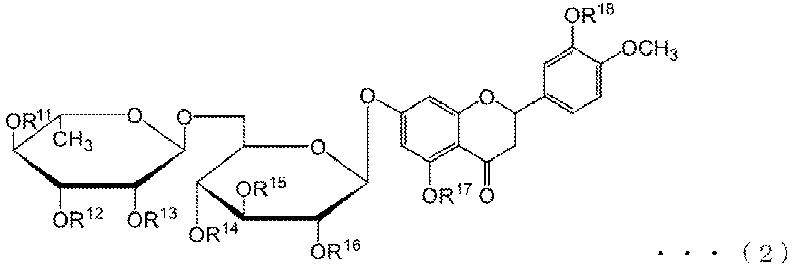
wherein in Formula (1), R¹ to R⁹ are each independently a methyl group or a hydrogen atom, provided that at least one of R¹ to R⁹ is a methyl group, and
in Formula (2), R¹¹ to R¹⁸ are each independently a methyl group or a hydrogen atom, provided that at least one of R¹¹ to R¹⁸ is a methyl group.

3. The barrier function improver according to Claim 2,
wherein the methyl hesperidin is one or more selected from the group consisting of chalcone form methyl hesperidin represented by General Formula (3) and flavanone form methyl hesperidin represented by General Formula (4), 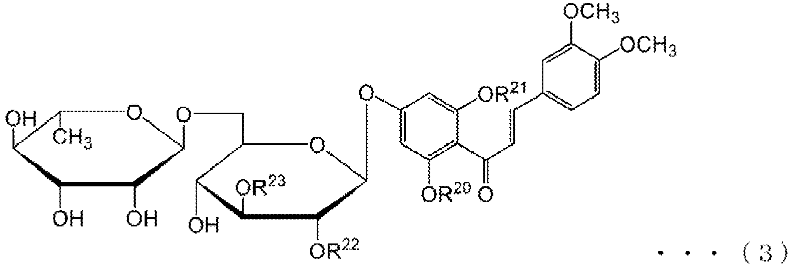 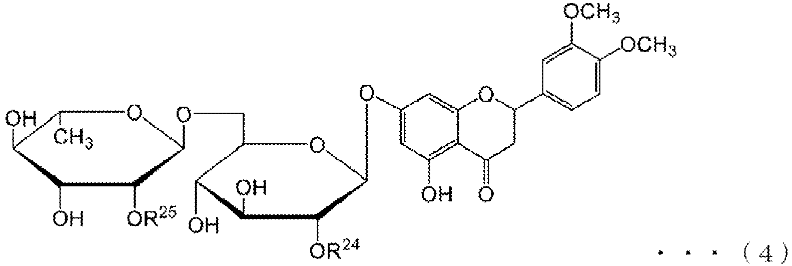
wherein in Formula (3), R²⁰ to R²³ are each independently a methyl group or a hydrogen atom, and
in Formula (4), R²⁴ and R²⁵ are each independently a methyl group or a hydrogen atom.

4. The barrier function improver according to Claim 3,
wherein the chalcone form methyl hesperidin represented by General Formula (3) is one or more selected from the group consisting of chalcone forms 1 to 3 having a combination of R²⁰ to R²³ shown in Table 1 below.
**[Table 1]**
| | R²⁰ | R²¹ | R22 | R²³ |
|---|---|---|---|---|
| Chalcone form 1 | CH₃ | CH₃ | CH₃ | CH₃ |
| Chalcone form 2 | H | CH₃ | CH₃ | H |
| Chalcone form 3 | H | CH₃ | H | H |

5. The barrier function improver according to Claim 3 or 4,
wherein the flavanone form methyl hesperidin represented by General Formula (4) is one or more selected from the group consisting of flavanone forms 1 to 4 having a combination of R²⁴ and R²⁵ shown in Table 2 below.
**[Table 2]**
| | R²⁴ | R²⁵ |
|---|---|---|
| Flavanone form 1 | CH₃ | CH₃ |
| Flavanone form 2 | CH₃ | H |
| Flavanone form 3 | H | H |
| Flavanone form 4 | H | CH₃ |

6. The barrier function improver according to any one of Claims 1 to 5,
wherein the barrier function improver is for promoting production of filaggrin.

7. The barrier function improver according to any one of Claims 1 to 5,
wherein the barrier function improver is for promoting production of involucrin.

8. The barrier function improver according to any one of Claims 1 to 5,
wherein the barrier function improver is for promoting production of loricrin.

9. The barrier function improver according to any one of Claims 1 to 5,
wherein the barrier function improver is for promoting production of transglutaminase 1.

10. The barrier function improver according to any one of Claims 1 to 5,
wherein the barrier function improver is for promoting production of aquaporin 3.

11. The barrier function improver according to any one of Claims 1 to 5,
wherein the barrier function improver is for promoting production of claudin 1.

12. A barrier-function-improving composition comprising:
the barrier function improver according to any one of Claims 1 to 11; and
a pharmaceutically acceptable carrier.

13. The barrier-function-improving composition according to Claim 12,
wherein a total content of the methyl hesperidin is 0.01% to 2% by mass.
